# EUROPÄISCHE PATENTSCHRIFT

(11) **EP 1 297 801 B1**
(45) Veröffentlichungstag und Bekanntmachung des Hinweises auf die Patenterteilung: **22.11.2006**
(21) Anmeldenummer: 02450208.0
(22) Anmeldetag: 18.09.2002
(51) Int. Cl.: A61F 4/00, G06F 3/00

(54) **Steuereinheit**
Control unit
Unité de Commande

(30) Priorität: 01.10.2001 AT 15532001
(43) Veröffentlichungstag der Anmeldung: 02.04.2003
(73) Patentinhaber: ARC Seibersdorf Research GmbH, 1010 Wien (AT); Diakonie Österreich, 1080 Wien (AT)
(72) Erfinder: Szivatz, Michael, 7053 Hornstein (AT); Fugger, Erwin, 2486 Pottendorf (AT); Burger, Thomas, 4221 Steyregg (AT); Gäbler, Gerhard, 4210 Gallneukirchen (AT)
(74) Vertreter: Wildhack, Helmut

(56) Entgegenhaltungen:
- EP-A- 0 854 437
- DE-U- 29 705 610
- ES-A- 2 078 853
- US-A- 5 460 186
- US-B1- 6 222 524

## Beschreibung

Die Erfindung betrifft eine Steuereinheit gemäß dem Oberbegriff des Patentanspruches 1.

Eine Steuereinheit gemäß dem Oberbegriff von Anspruch 1 ist beispielsweise aus der ES-A-2 078 853 bekannt.

Es sind Geräte bekannt, die es Menschen mit spezifischen Behinderungen, insbesondere bei hoher Querschnittslähmung, erlauben, einen Computer und/oder andere elektronische Geräte mittels eines mundgesteuerten Mausersatzes (Mundmaus) zu bedienen. Derartige Steuereinheiten sind oftmals die einzige Möglichkeit, mit denen gelähmte Menschen Tätigkeiten verrichten bzw. kommunizieren können. Derartige Steuereinheiten ermöglichen auch die Nutzung von Softwaretools zu Therapiezwecken, die Einflussnahme auf die Umwelt oder den Zugang zum Internet, womit eine Verbesserung der Lebensqualität für die Benutzer erreicht bzw. die Arbeit von Therapeuten und Betreuern erleichtert werden kann.

Insbesondere soll eine derartige Steuereinheit ein elektronisches Hilfsmittel darstellen, das Menschen mit fehlender Funktion ihrer Arme und Hände ermöglichen soll, einen Computer bzw. andere elektronische Geräte zu bedienen, insbesondere um Texte zu schreiben bzw. Schalt- oder Steuerungsvorgänge in ihrer Umgebung auszulösen.

Eine derartige Steuereinheit soll einfach und betriebssicher aufgebaut sein, beträchtlichen Beanspruchungen standhalten und vor allem exakte Signale liefern.

Erfindungsgemäß ist eine Steuereinheit der eingangs genannten Art mit den im Kennzeichen des Anspruches 1 angeführten Merkmalen charakterisiert.

Eine derart aufgebaute Steuereinheit ist einfach und kostengünstig zu erstellen. Durch den vorgegebenen Aufbau erfolgt eine exakte Umsetzung der Bewegungen des mundbetätigbaren Stellteiles in Ausgangssignale, die entsprechende Steuervorgänge auslösen können. Die erfindungsgemäße Steuereinheit kann gemäß den Vorschriften für Medizinprodukte ausgebildet werden, welchen Vorschriften Genüge geleistet werden soll, wenn die Steuereinheit zur Kompensation einer Behinderung eingesetzt werden soll.

Bei einer bevorzugten Ausführungsform der Erfindung sind die Merkmale des Anspruches 6 oder des Anspruches 8 verwirklicht, sodass voneinander unabhängige Steuervorgänge eingeleitet werden können. Es kann somit nicht nur die Funktion eines Mauszeigers, sondern auch die Funktion eines Mausklicks mit der erfindungsgemäßen Steuereinheit erreicht werden.

Weitere vorteilhafte Ausführungsformen der Erfindung ergeben sich aus der folgenden Beschreibung und aus den Patentansprüchen.

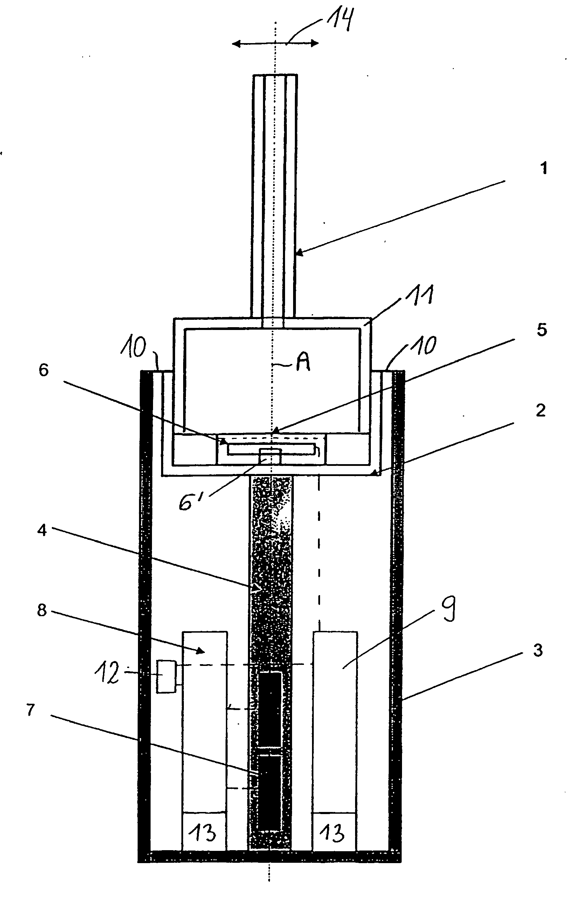

Im Folgenden wird die Erfindung anhand der Zeichnung näher beschrieben, in der ein schematisches Ausführungsbeispiel einer erfindungsgemäßen Steuereinheit beschrieben wird.

Die erfindungsgemäße Steuereinheit umfasst als Mundstück einen rohrförmigen Stellteil 1, der vom Benutzer mit den Zähnen und/oder den Lippen verstellt werden kann.

Die Aufnahme 2 ist einstückig oder verbunden, gegebenenfalls verklebt, mit einem elastisch biegbaren Stab 4, dessen anderes Ende insbesondere im Bodenbereich des Gehäuses 3 festgelegt bzw. befestigt ist. Die Aufnahme 2 ist auf dem Stab 4 relativ zum starren Gehäuse 3, insbesondere in alle Richtungen einer Ebene senkrecht zur Längsachse des Stellteiles 1 schwenkbar gelagert. Damit ist es möglich, bei Kraftausübung auf das Ende des Stellteiles 1 die Aufnahme 2 zu verschwenken, welche Verschwenkung wiederum eine Verbiegung des Stabes 4 zur Folge hat.

Der Stellteil 1 kann in Richtung des Pfeiles 14 verstellt werden. Da es sich bei der Zeichnung um eine zweidimensionale Darstellung handelt, ist der Pfeil 14 derart zu verstehen, dass der Stellteil 1 auch in Richtungen senkrecht und/oder im Winkel zur Zeichnungsebene verstellt werden kann. Insbesondere kann der Stellteil 1 in beliebige Richtungen in einer Ebene senkrecht zu seiner Achse A vom Benutzer verstellt werden. Vorteilhaft handelt es sich bei dem Stellteil 1, bei dem an den Stellteil anschließenden Kammerteil 11, bei der mit dem Kammerteil 11 verbundene Aufnahme 2 und bei dem mit der Aufnahme 2 verbundenen Stab 4 um eine rotationssymmetrische Anordnung handelt, die Verstell- bzw. Verschwenkbewegungen in beliebige Raumrichtungen zulässt. Die durch derartige Bewegungen hervorgerufene Biegung des Stabes 4 wird abgefühlt.

Auf dem Stab 4 sind Dehnmess-Streifen 7 aufgeklebt, mit denen die Biegung des Stabes 4 aufgenommen wird bzw. die von der Biegung abhängige Ausgangssignale abgeben. Die Dehnmess-Streifen 7 reagieren auf eine Verkürzung bzw. Verlängerung der Oberfläche des sich biegenden Stabes 4 und werden in Längsrichtung des Stabes 4, insbesondere um den Umfang des Stabes 4 herum, vorzugsweise versetzt, angeordnet. Es können mehrere Dehnungsmess-Streifen 7 um den Umfang des Stabes 4, z.B. um 90° zueinander, versetzt und/oder in Längsrichtung des Stabes 4, gegebenenfalls versetzt zueinander, angeordnet werden. Vorteilhaft ist es, wenn vier Dehnmess-Streifen 7 um den Umfang des Stabes 4 herum, jeweils um 90° zueinander versetzt, angeordnet werden, um eine definierte Geometrie zu erreichen.

Zur Auswertung der von den Dehnmess-Streifen 7 abgegebenen Signale ist eine elektronische Schaltung 8 vorgesehen.

Die durch selektive Dehnung der einzelnen Dehnmess-Streifen 7 bei Verbiegung des Stabes 4 eintretende Widerstandsänderung wird von der elektronischen Schaltung 8 abgefühlt, gegebenenfalls verstärkt und in Ausgangssignale umgewandelt, welche für die Steuerung von Schaltvorgängen, z.B. eines Computers, insbesondere zur Positionierung des Mauszeigers, genutzt werden können. Abhängig von der Bewegungsrichtung des Stellteiles 1 aufgrund der in den Stellteil 1 eingeleiteten Kraft kann der Mauszeiger vom Benutzer gezielt über den Bildschirm bewegt werden.

Der Stellteil 1 ist mit einem Kammerteil 11 verbunden, der becherförmig ausgestaltet ist. Das Innere des Stellteils 1 und das Innere des Kammerteiles 11 kommunizieren. Der Kammerteil 11 ist an seinem, in der Zeichnung unteren, Ende mit einer Membran 5 abgeschlossen, die an einem druckempfindlichen Widerstand 6 unter Vorspannung anliegt. Der Kammerteil 11 ist von einer Aufnahme 2 aufgenommen, insbesondere in diese Aufnahme eingesteckt bzw. eingeschraubt. Durch das Einstecken bzw. Einschrauben kann der Anpressdruck der Membran 5 an dem druckempfindlichen Widerstand 6 eingestellt werden. Der Widerstand 6 kann am Boden der becherförmigen Aufnahme 2 angeklebt sein.

Durch Blasen oder Saugen in den bzw. am Stellteil 1 entsteht an der Membran 5 ein Über- oder Unterdruck, und ein entsprechendes Drucksignal kann an dem druckempfindlichen Widerstand 6 abgenommen werden. Die entstehenden Widerstandsänderungen werden von einer elektronischen Schaltung 9 abgefühlt und ausgewertet und für Stell- bzw. Schaltvorgänge, z.B. zur Auslösung eines Mausklicks genutzt. Anstelle des Widerstandes 6 kann eine optische Abstandmesseinrichtung 6' treten, wie sie in der Zeichnung angedeutet ist. Die Abstandmesseinrichtung 6' misst die Distanz zur Membran 5; bei Veränderung des Abstandes bzw. einer Auslenkung der Membran 5 durch Blasen oder Saugen wird bei Überschreiten von vorgegebenen Werten ein Ausgangs- bzw. Schaltsignal abgegeben, das in der elektronischen Schaltung 9 in gleicher Weise ausgewertet bzw. weitergegeben wird, wie das Schaltsignal des druckempfindlichen Widerstandes 6.

Eine derartige Abstandmesseinrichtung 6' kann z.B. eine Sende-Empfangseinheit für IR-Strahlung umfassen, wobei die IR-Strahlung von der Membran 5 in den Empfänger reflektiert wird und Abstandänderungen detektiert werden.

Die Ausgangssignale der Schaltungen 8 und/oder 9 können allenfalls mit einem Sender 13 an einen Schaltvorgänge auslösenden Empfänger, z.B. Computer, übertragen werden.

Mit 12 ist eine Stromversorgung für die elektronischen Schaltungen 8 und 9 bzw. für die Dehnmess-Streifen 7 und den Widerstand 6 oder die optische Abstandmesseinrichtung 6' angedeutet.

Mit Softwaretools können Feineinstellungen für den Kraftaufwand und/oder die Geschwindigkeit vorgenommen werden, mit der die auf den Stellteil 1 bzw. auf den Widerstand 6 ausgeübten Kräfte bzw. Drucke in entsprechenden Schaltvorgänge umgesetzt werden, insbesondere in Bewegungen des Mauszeigers bzw. vorgegebene Durchführung des Mausklicks. Dabei ist auch die Elastizität bzw. der Biegewiderstand des Stabes 4 sowie die Auslenkcharakteristik der Membran 5 zu berücksichtigen, um die auf den Stellteil 1 bzw. die Membran 5 auszuübenden Kräfte nicht übermäßig groß werden zu lassen bzw. auf einem gewünschten Bereich zu beschränken.

Vorteilhafter Weise besteht das Mundstück bzw. der Stellteil 1 aus gewebeverträglichem Kunststoff. Die Membran 5 soll aus hygienischen Gründen auf dem Kammerteil 11 auswechselbar gelagert sein. Diese auswechselbare Lagerung könnten z.B. darin bestehen, dass die Membran 5 von einem Ring getragen ist, wobei der Ring in den Kammerteil 11 dicht einschraubbar oder einsteckbar ist.

Des Weiteren ist der Kammerteil 11 mit dem Stellteil 1 austauschbar in der Aufnahme 2 gelagert; es ist somit möglich, den Kammerteil 11, die Membran 5 und den Stellteil 1 auszuwechseln bzw. abzunehmen und zu desinfizieren, um verschiedenen Benutzern entsprechende hygienische Voraussetzungen zu bieten.

Da die Steuereinheit ohne Funktionseinschränkungen mit einer Funkdatenübertragung bzw. mit autonomer Stromversorgung ausgestattet werden kann, so können auch mobile Benutzer, die an einen Rollstuhl gebunden sind, diese Steuereinheit benutzen.

Die elektronischen Schaltungen 8 und 9 können auch zu einer einzigen Schaltung integriert sein, die einen Mikroprozessor umfasst. Schaltungen zur Auswertung der an den Dehnmess-Streifen 7 anliegenden Widerstandswerte sind dem Fachmann geläufig.

Die Ausbildung und Wahl der Trägereinheiten 10 für die Aufnahme 2 wird derart getroffen, dass die Aufnahme 2 einer Verbiegung des Stabes 4 bei Betätigung des Stellteiles 1 nicht allzu großen Widerstand entgegensetzt bzw. dass die Kräfte der Trägereinheit 10 mit dem Stellteil 1 relativ leicht zu überwinden sind. Derartige Trägereinheiten 10 können z.B. durch gummielastische Bauteile verwirklicht werden, da die auf den Stab 4 für seine Abbiegung auszuübenden Kräfte relativ gering gehalten werden können.

## Patentansprüche

1. Steuereinheit, insbesondere Maus- und/oder Tastaturersatz, mit einem mundbetätigbaren Stellteil (1), der von einem Gehäuse ausragt und relativ zu dem starren Gehäuse (3) kipp- bzw. verschwenkbar im Gehäuse (3) gelagert ist, und mit einem, mit dem Stellteil (1) beeinflussbaren, in dem Gehäuse (3) angeordneten Steuerteil (4), **dadurch gekennzeichnet,**
- **dass** der Stellteil (1) von einem Rohr gebildet ist und mit einer Aufnahme bzw. einem Zwischenstück (2), insbesondere durch Stecken oder Schrauben, verbunden ist,
- **dass** die Aufnahme (2) mit dem einen Ende eines als Steuerteil fungierenden, elastisch biegbaren Stabes (4) verbunden ist, dessen anderes Ende am vorzugsweise zylindrischen Gehäuse (3), insbesondere in dessen Bodenbereich, festgelegt bzw. befestigt ist,
- **dass** am Stab (4), insbesondere in dessen gehäusenahen Endbereich, zumindest ein, vorzugsweise vier oder acht, Dehnmess-Streifen (7) befestigt ist bzw. sind, und
- **dass** zum Abfühlen der sich bei Verschwenkung des Steuerteils (1) verändernden Widerstandswerte des bzw. der Dehnmess-Streifen (7) - wie an sich bekannt - eine elektronische Schaltung (8) vorgesehen ist, mit der die abgefühlten Werte in elektrische Ausgangssignale, insbesondere zur Steuerung der Positionierung eines Mauszeigers, umsetzbar sind.

2. Steuereinheit nach Anspruch 1, **dadurch gekennzeichnet, dass** über den Umfang des Stabes (4), insbesondere auf gleicher Höhe, zumindest zwei Dehnmess-Streifen (7), vorzugsweise vier Dehnmess-Streifen (7), um 90° zueinander versetzt angeordnet sind.

3. Steuereinheit nach Anspruch 1 oder 2, **dadurch gekennzeichnet, dass** zwischen der Aufnahme (2) und dem, vorzugsweise langgestreckten, Gehäuse (3) ein Abstand oder ein Spalt (10) ausgebildet ist.

4. Steuereinheit nach einem der Ansprüche 1 bis 3, **dadurch gekennzeichnet, dass** der Stab (4) und die Aufnahme (2) einstückig oder durch Verschrauben und/oder Verkleben miteinander verbunden sind und vorzugsweise aus Kunststoff oder Metall bestehen.

5. Steuereinheit nach einem der Ansprüche 1 bis 4, **dadurch gekennzeichnet, dass** mit der elektronischen Schaltung (8) die Schnelligkeit der Verstellung und/oder die Richtung der Auslenkung des Steuerteiles (1), basierend auf den sich zeitlich verändernden Widerstandeswerten der Dehnmess-Streifen (7) feststellbar ist, wobei vorzugsweise die elektrischen Ausgangssignale der Schaltung (8) der Schnelligkeit und/oder der Richtung der Auslenkung direkt oder indirekt proportional sind.

6. Steuereinheit nach einem der Ansprüche 1 bis 5, **dadurch gekennzeichnet, dass** der rohrförmige Stellteil (1) gegebenenfalls einstückig oder durch Schrauben, Stecken od.dgl. mit einem Kammerteil (11) ausgebildet oder verbunden ist, welcher Kammerteil (11) mit der Aufnahme (2) verbindbar ist, gegebenenfalls in bzw. auf diese ein- bzw. aufsteckbar oder ein- bzw. aufschraubbar ist, wobei der Innenraum des luftdurchgängig ausgebildeten Stellteiles (1) und das Innere des Kammerteiles (11) kommunizieren,
- dass von der Aufnahme (2), insbesondere deren inneren Basisfläche, ein druckempfindlicher Widerstand (6) getragen ist,
- dass der der Aufnahme (2) nahe Endbereich des Kammerteiles (11) mit einer durch Blasen oder Saugen durch den Stellteil (1) auslenkbaren Membran (5), insbesondere dicht, abgeschlossen ist,
- dass die Membran (5) mit einer vorgegebenen Vorspannung, vorzugsweise direkt, an dem druckempfindlichen Widerstand (6) angelegt bzw. an diesem angedrückt ist, und
- dass eine elektronische Steuerung (9) vorgesehen ist, mit der die druckabhängigen Signale des Widerstandes (6) in elektrische Ausgangssignale , insbesondere zur Auslösung eines Mausklicks, umsetzbar sind.

7. Steuereinheit nach einem der Ansprüche 1 bis 6, **dadurch gekennzeichnet, dass** die mit der Membran (5) abgeschlossene Fläche bzw. lichte Weite des Kammerteiles (11) die lichte Weite des Stellteiles (1) um ein Vielfaches, vorzugsweise um das 20- bis 100-fache, übersteigt.

8. Steuereinheit nach einem der Ansprüche 1 bis 7, **dadurch gekennzeichnet, dass** der rohrförmige Stellteil (1) gegebenenfalls einstückig oder durch Schrauben, Stecken od.dgl. mit einem Kammerteil (11) ausgebildet oder verbunden ist, welcher Kammerteil (11) mit der Aufnahme (2) verbindbar ist, gegebenenfalls in bzw. auf diese ein- bzw. aufsteckbar oder ein- bzw. aufschraubbar ist, wobei der Innenraum des luftdurchgängig ausgebildeten Stellteiles (1) und das Innere des Kammerteiles (11) kommunizieren,
- dass der der Aufnahme (2) nahe Endbereich des Kammerteiles (11) mit einer durch Blasen oder Saugen durch den Stellteil (1) auslenkbaren Membran (5), insbesondere dicht, abgeschlossen ist,
- dass von der Aufnahme (2), insbesondere deren inneren Basisfläche, im Abstand zur Membran (5) ein optisches Abstandmessgerät (6') getragen ist, und
- dass eine elektronische Steuerung (9) vorgesehen ist, mit der die abstandabhängigen Signale des Abstandmessgerätes (6') in elektrische Ausgangssignale , insbesondere zur Auslösung eines Mausklicks, umsetzbar sind.

9. Steuereinheit nach einem der Ansprüche 1 bis 8, **dadurch gekennzeichnet, dass** der Stellteil (1) aus gewebeverträglichem, desinfizierbarem bzw. sterilisierbarem Material, vorzugsweise Kunststoff, ausgebildet ist.

10. Steuereinheit nach einem der Ansprüche 1 bis 9, **dadurch gekennzeichnet, dass** die Membran (5) auf dem Kammerteil (11) auswechselbar gelagert ist, wozu gegebenenfalls die Membran (5) von einem am Kammerteil (11) auf- bzw. einschraubbaren bzw. ein- bzw. aufsteckbaren Ring getragen ist.

11. Steuereinheit nach einem der Ansprüche 1 bis 10, **dadurch gekennzeichnet, dass** die elektrische (n) Schaltung (en) (8,9) die Ausgangssignale der Dehnmess-Streifen (7) und/oder des druckempfindlichen Widerstandes (6) oder des Abstandmessgerätes (6') in Form von Funksignalen zur Verfügung stellen bzw. dass an die Schaltungen (8,9) ein Sender (13) für diese Signale angeschlossen ist.

12. Steuereinheit nach einem der Ansprüche 1 bis 11, **dadurch gekennzeichnet, dass** die Stromversorgung (12), z.B. Batterien, in das Gehäuse (3) integriert sind.

13. Steuereinheit nach einem der Ansprüche 1 bis 12, **dadurch gekennzeichnet, dass** die elektronischen Schaltungen (8,9) mit Softwaretools zur Feinanpassung der Auslenkung des Stellteiles (1) und/oder der Kraftbeaufschlagung oder Auslenkung der Membran (5) an die gewünschte Positionierung des Mauszeigers und/oder den gewünschten Auslösedruck für einen Mausklick gesteuert sind.

14. Steuereinheit nach einem der Ansprüche 1 bis 13, **dadurch gekennzeichnet, dass** die Aufnahme (2) und/oder der Kammerteil (11) und/oder der Stab (4) und/oder der Stellteil (1) kreisförmigen Außen- und/oder Innenquerschnitt aufweisen.

15. Steuereinheit nach einem der Ansprüche 1 bis 14, **dadurch gekennzeichnet, dass** der Stellteil (1) und der Stab (4) dieselbe Längsachse (A) besitzen.

16. Steuereinheit nach einem der Ansprüche 1 bis 15, **dadurch gekennzeichnet, dass** die Steifigkeit des Stellteiles (1) größer ist als die des Stabes (4).

## Claims

1. Control unit, particularly mouse and/or keyboard substitute, comprising a mouth actuable setting part (1), which projects from a housing and is tiltably or pivotally supported in said housing (3) relative to the rigid housing (3), and having a control part (4) being able to be influenced by said setting part (1) and located in said housing (3), **characterised in**
- **that** said setting part (1) is formed by a pipe and is connected to a holding fixture or intermediate piece (2), particularly by plugging or screwing,
- **that** said holding fixture (2) is connected to one end of an elastically flexible rod (4) functioning as a control part, the other end of which is fixed or fastened to the, preferably cylindrical, housing (3), particularly in its bottom region,
- **that** at least one wire strain gauge (7), preferably four or eight, is or are fastened to said rod (4), particularly in its end region near the housing, and
- **that** an electronic circuit (8) is provided, as is known per se, for sensing the resistance values of the wire strain gauge or gauges (7), that change when pivoting said control part (1), and by which the sensed values may be transformed into electric output signals, particularly for controlling positioning of a mouse indicator.

2. Control unit according to claim 1, **characterised in that** at least two wire strain gauges (7), preferably four wire strain gauges (7), are arranged offset by 90° to one another over the circumference of the rod (4), particularly at the same level.

3. Control unit according to claim 1 or 2, **characterised in that** a distance or gap (10) is formed between the holding fixture (2) and the, preferably elongated, housing (3).

4. Control unit according to any of claims 1 to 3, **characterised in that** the rod (4) and the holding fixture (2) are integral or interconnected by screwing and/or cementing, and consist preferably of plastic material or of metal.

5. Control unit according to any of claims 1 to 4, **characterised in that** the speed of displacement and/or the direction of deflection of said control part (1) is detectable by said electronic circuit (8) based on the resistance values of the wire strain gauges (7), that vary in time, the electric output signals of said circuit (8) being preferably directly or indirectly proportional to the speed and/or to the direction of deflection.

6. Control unit according to any of claims 1 to 5, **characterised in that** the tubular setting part (1) is optionally formed integrally with or is connected by screwing, plugging or the like to a chamber part (11), said chamber part (11) being connectable to said holding fixture (2), and may optionally be plugged into or onto it or may be screwed into or onto it, the inner space of the air conductive setting part (1) and the interior of said chamber part (11) being in communication,
- that a pressure sensitive resistance (6) is born by said holding fixture (2), particularly by its inner base surface,
- that the terminal region of said chamber part (11) near to said holding fixture (2) is closed, particularly tightly, by a membrane (5), which may be deflected by blowing or sucking through said setting part (1),
- that the membrane (5) is engaging or is pressed, preferably directly, onto said pressure sensitive resistance (6) with a predetermined bias, and
- that an electronic control (9) is provided, by which the pressure dependent signals of said resistance (6) may be transformed into electric output signals, particularly for releasing a mouse click.

7. Control unit according to any of claims 1 to 6, **characterised in that** the surface closed by the membrane (5) or the inside width of said chamber part (11) exceeds the inside width of said setting part (1) by a multiple, preferably by the 20- to 100-fold.

8. Control unit according to any of claims 1 to 7, **characterised in that** the tubular setting part (1) is optionally formed integrally with or is connected by screwing, plugging or the like to a chamber part (11), said chamber part (11) being connectable to said holding fixture (2), and may optionally be plugged into or onto it or may be screwed into or onto it, the inner space of the air conductive setting part (1) and the interior of said chamber part (11) being in communication,
- that the terminal region of said chamber part (11) near to said holding fixture (2) is closed, particularly tightly, by a membrane (5), which may be deflected by blowing or sucking through said setting part (1),
- that an optical distance gauge instrument (6') is born by said holding fixture (2), particularly by its inner base surface, in a distance to said membrane (5), and
- that an electronic control (9) is provided, by which the distance dependent signals of said distance gauge instrument (6') may be transformed into electric output signals, particularly for releasing a mouse click.

9. Control unit according to any of claims 1 to 8, **characterised in that** the setting part (1) is formed of a tissue compatible material, preferably a plastic material, that may be disinfected or sterilised.

10. Control unit according to any of claims 1 to 9, **characterised in that** the membrane (5) is exchangeably supported on said chamber part (11), to which end said membrane (5) is optionally supported by a ring, which may be screwed into or onto or may be plugged into or onto said chamber part (11).

11. Control unit according to any of claims 1 to 10, **characterised in that** the electric circuit(s) (8, 9) put the output signals of said wire strain gauges (7) and/or of said pressure sensitive resistance (6) or of the distance gauge instrument (6') at disposal in the form of radio signals and/or that a transmitter (13) is coupled to said circuits (8, 9).

12. Control unit according to any of claims 1 to 11, **characterised in that** the power supply (12), e.g. batteries, are integrated into said housing (3).

13. Control unit according to any of claims 1 to 12, **characterised in that** the electronic circuits (8, 9) are controlled by software tools for finely adapting the deflection of said setting part (1) and/or the bias force or deflection of said membrane (5) to the desired positioning of the mouse indicator and/or the desired release pressure for a mouse click.

14. Control unit according to any of claims 1 to 13, **characterised in that** the holding fixture (2) and/or the chamber part (11) and/or the rod (4) and/or the setting part (1) have a circular outer and/or inner cross-section.

15. Control unit according to any of claims 1 to 14, **characterised in that** the setting part (1) and the rod (4) have the same longitudinal axis (A).

16. Control unit according to any of claims 1 to 15, **characterised in that** rigidity of the setting part (1) is greater than that of said rod (4).

## Revendications

1. Unité de commande, particulièrement produit de substitution de souris et/ou de touches, comprenant une partie de réglage (1) manoeuvrable à bouche, qui fait saillie d'un boîtier et est logée dans le boîtier (3) d'une façon basculante ou pivotante relativement au boîtier (3) rigide, et une partie de commande (4) influencée par la partie de réglage (1) et disposée dans le boîtier (3), **caractérisée en ce**,
- la partie de réglage (1) est formée par un tuyau et est reliée à un porte-pièce ou une pièce intercalaire (2), particulièrement par enfichage ou vissage,
- que le porte-pièce (2) est relié à un bout d'une barre (4) élastiquement flexible, qui a la fonction d'une partie de commande, dont l'autre bout est fixé ou monté au boîtier (3), préférablement cylindrique, particulièrement à la zone du fond de ceci,
- qu'au moins une jauge extensométrique (7), de préférence quatre ou huit, est ou sont attaché à la barre (4), particulièrement dans sa zone terminale proche au boîtier, et
- qu'un circuit électronique (8) est prévu, comme connu en soi, pour détecter les valeurs de résistance de la (des) jauge(s) extensométrique(s) (7), qui changent lors du pivotement de la partie de réglage (1), par lequel les valeurs détectés peuvent être transformés en signaux de sortie électriques, particulièrement pour commander le positionnement d'un indicateur de souris.

2. Unité de commande selon la revendication 1, **caractérisée en ce, qu'**au moins deux jauges extensométriques (7), de préférence quatre jauges extensométriques (7), sont disposées d'une façon décalée l'une à l'autre par 90° à la circonférence de la barre (4), particulièrement au même niveau.

3. Unité de commande selon la revendication 1 ou 2, **caractérisée en ce, qu'**un écartement ou une fente (10) est formé entre le porte-pièce (2) et le boîtier (3), qui est de préférence allongé.

4. Unité de commande selon une quelconque des revendications 1 à 3, **caractérisée en ce, que** la barre (4) et le porte-pièce (2) sont reliés l'un à l'autre soit d'une seule partie, soit par vissage et/ou par collage, et qu'ils consistent préférablement d'une matière plastique ou d'un métal.

5. Unité de commande selon une quelconque des revendications 1 à 4, **caractérisée en ce, que**, fondé sur les valeurs de résistance des jauges extensométriques (7), qui changent en fonction du temps, on peut déterminer avec le circuit électronique (8) la vitesse du déplacement et/ou la direction de l'excursion de la partie de réglage (1), les signaux de sortie électriques du circuit (8) étant de préférence directement ou indirectement proportionnels à la vitesse et/ou à la direction de l'excursion.

6. Unité de commande selon une quelconque des revendications 1 à 5, **caractérisée en ce, que** la partie de réglage (1) tubulaire est, le cas échéant, est formée d'une seule partie avec une partie de chambre (11) ou est reliée à elle par vissage, par enfichage ou pareil, ladite partie de chambre (11) pouvant être reliée au porte-pièce (2) en étant, le cas échéant, enfichable ou embrochable dans ceci ou à ceci, ou bien étant capable d'être vissée dans ou au ceci, dans laquelle l'espace intérieur de la partie de réglage (1) faisant conduite à l'air est en communication avec l'intérieur de la partie de chambre (2),
- qu'une résistance (6) sensible à la pression est portée par le porte-pièce (2), particulièrement par sa surface interne de base,
- que la zone terminale de la partie de chambre (11), proche au porte-pièce (2), est fermée, particulièrement d'une façon étanche, par une membrane (5), qui peut subir une excursion par soufflage ou par aspiration à travers la partie de réglage (1),
- que la membrane (5) est en contact ou est pressée, de préférence directement, avec une précontrainte prédéterminée contre la résistance (6) sensible à la pression, et
- qu'une commande électronique (9) est prévue, par laquelle les signaux en fonction de la pression de la résistance (6) peuvent être transformés en signaux de sortie électriques, particulièrement pour déclencher un clic de souris.

7. Unité de commande selon une quelconque des revendications 1 à 6, **caractérisée en ce, que** la surface fermée par la membrane (5) ou la largeur intérieur de la partie de chambre (11) dépasse la largeur intérieur de la partie de réglage (1) par un multiple, préférablement par le vingtuple au centuple.

8. Unité de commande selon une quelconque des revendications 1 à 7, **caractérisée en ce, que** la partie de réglage (1) tubulaire est, le cas échéant, est formée d'une seule partie avec une partie de chambre (11) ou est reliée à elle par vissage, par enfichage ou pareil, ladite partie de chambre (11) pouvant être reliée au porte-pièce (2) en étant, le cas échéant, enfichable ou embrochable dans ceci ou à ceci, ou bien étant capable d'être vissée dans ou au ceci, dans laquelle l'espace intérieur de la partie de réglage (1) faisant conduite à l'air est en communication avec l'intérieur de la partie de chambre (2),
- que la zone terminale de la partie de chambre (11), proche au porte-pièce (2), est fermée, particulièrement d'une façon étanche, par une membrane (5), qui peut subir une excursion par soufflage ou par aspiration à travers la partie de réglage (1),
- qu'un appareil écartométrique (6') est portée par le porte-pièce (2), particulièrement par sa surface interne de base, en écart à la membrane (5), et
- qu'une commande électronique (9) est prévue, par laquelle les signaux de l'appareil écartométrique (6') en fonction de l'écart peuvent être transformés en signaux de sortie électriques, particulièrement pour déclencher un clic de souris.

9. Unité de commande selon une quelconque des revendications 1 à 8, **caractérisée en ce, que** la partie de réglage (1) est formé d'une matière compatible aux tissues et capable à être désinfecté ou d'être stérilisé, préférablement d'une matière plastique.

10. Unité de commande selon une quelconque des revendications 1 à 9, **caractérisée en ce, que** la membrane (5) est logée à la partie de chambre (11) d'une manière échangeable, auquel effet, le cas échéant, la membrane (5) est portée d'un anneau, qui peut être vissé ou enfiché dans la ou à la partie de chambre (11).

11. Unité de commande selon une quelconque des revendications 1 à 10, **caractérisée en ce, que** le (les) circuit(s) électrique(s) (8, 9) met(tent) à la disposition les signaux de sortie des jauges extensométriques (7) et/ou de la résistance (6) sensible à la pression ou de l'appareil écartométrique (6') en forme des signaux radio et/ou qu'un poste émetteur (13) pour ces signaux est relié à ces circuits (8, 9).

12. Unité de commande selon une quelconque des revendications 1 à 11, **caractérisée en ce, que** l'alimentation en courant (12), par exemple des batteries, est intégrée dans le boîtier (3).

13. Unité de commande selon une quelconque des revendications 1 à 12, **caractérisée en ce, que** les circuits électroniques (8, 9) sont commandés par des outils de logiciel pour l'adaptation fine de l'excursion de la partie de réglage (1) et/ou de la charge à force ou l'excursion de la membrane (5) au positionnement souhaité de l'indicateur de souris et/ou à la pression souhaitée de déclenchement pour un clic de souris.

14. Unité de commande selon une quelconque des revendications 1 à 13, **caractérisée en ce, que** le porte-pièce (2) et/ou la partie de chambre (11) et/ou la barre (4) et/ou la partie de réglage (1) présentent une coupe transversale circulaire externe et/ou interne.

15. Unité de commande selon une quelconque des revendications 1 à 14, **caractérisée en ce, que** la partie de réglage (1) et la barre (4) ont le même axe longitudinal (A).

16. Unité de commande selon une quelconque des revendications 1 à 15, **caractérisée en ce, que** la rigidité de la partie de réglage (1) est plus grande que celle de la barre (4).
